# EUROPEAN PATENT APPLICATION

(11) **EP 1 374 924 A1**
(43) Date of publication of application: **02.01.2004**
(21) Application number: 03013813.5
(22) Date of filing: 18.06.2003
(51) Int. Cl.: A61L 31/10, A61L 27/34, C08L 89/06, C08L 1/00, C08L 5/04, C08L 5/08

(54) **Coatings of implants**

(30) Priority: 18.06.2002 US 389551 P
(71) Applicant: Collagen Matrix, Inc., Franklin Lakes, NJ 07869 (US)
(72) Inventor: Li, Shu-Tung, Ph.D., Oakland, NJ 07436 (US); Yuen, Debbie, Woodcliff Lake, NJ 07677 (US)
(74) Representative: Brandenburg, Thomas, Dr.

(57) **Abstract**

An implant containing a support member and a biopolymer-based material formed integrally on a surface of the support member. The support member is dimensionally manipulatable, and the integrity of the biopolymer-based material is independent of dimensional changes in the support member. Also disclosed is a method of preparing such an implant.

## Description

### BACKGROUND

An implant with specific surface properties is crucial for its function. Vascular implants must have a blood-compatible surface in order to function in blood-contacting applications. Coating a support member with a bioactive agent such as an antibiotic can prevent infection. A support member coated with a therapeutic agent can function as a local drug delivery vehicle. Coating a support member with an anti-proliferative agent prevents proliferation of certain type of cells such as smooth muscle cells in the stenting of a blood vessel, coating a support member with a collagenous material promotes stable fibrous tissue formation in the treatment of cerebral aneurysms, and coating a support member with a hydrogel provides an implant with lubricious properties to prevent adhesion or improve the delivery of the implant such as a catheter.

### SUMMARY

The present invention features an implant that contains a support member, and a biopolymer-based material formed integrally on the surface of the support member. The support member is dimensionally manipulatable, and the integrity of the biopolymer-based material is independent of dimensional changes in the support member.

A "biopolymer-based material" can contain a biopolymer (e.g., protein such as collagen, or polysaccharide such as cellulose, chitosan, alginic acid, or glycosaminoglycan) alone, or be a mixture of a biopolymer and a synthetic polymer (e.g., polyglycolic acid, polylactic acid, polyglycolic acid/poly-L-lactic acid copolymers, polycaprolactive, polyhydroxybutyrate/hydroxyvalerate copolymers, polydioxanone, polycarbonates, polyanhydrides, polytetrathalate, and polytetrafluoroethylene).

In one example, the implant of the invention further contains a synthetic polymer formed integrally on an inner surface of the biopolymer-based material. In another example, the implant of the invention further contains a synthetic polymer formed integrally on an outer surface of the biopolymer-based material. A bioactive agent can be admixed with a biopolymer-based material or a synthetic polymer for therapeutic or other applications. When a synthetic polymer is formed on an inner surface of the biopolymer-based material, a bioactive agent admixed with the synthetic polymer can be delivered, yet the surface of the implant maintains its properties derived from the biopolymer-based material. When a synthetic polymer is formed on an outer surface of the biolpolymer-based material, the synthetic polymer can prevent the swelling of the biopolymer-based material, and thus provide a slower release of a bioactive agent that is admixed with the biopolymer-based material. Furthermore, in certain applications such as coils for aneurysm embolization, the degradation of synthetic polymers such as polyglycolic acid, polylactic acid or polyglycolic acid/poly-L-lactic acid copolymers on the outer layer into glycolic acid and lactic acid can promote active cellular responses, while the inner biolpolymer-based material provides a biosurface for cell adhesion, proliferation and matrix synthesis, e.g., fibrogenesis for stable occlusion of aneurysm.

"Formed integrally" refers to mechanical interactions (e.g., surface texture), physico-chemical characteristics (e.g., surface energy), and chemical interactions (e.g., hydrogen bonding and electrostatic interactions) between a biopolymer-based material and a support member, a biopolymer-based material and a synthetic polymer, or a synthetic polymer and a support member, resulting in a polymeric coating that does not fall off from the support member during the intended use of the implant. For example, coating of a biopolymer-based material on a metal stent results in a coating formed integrally on the surface of the stent predominantly via the mechanical and physico-chemical interactions, whereas coating of a biopolymer-based material on a flexible porous support member results in a coating formed integrally on the surface predominantly via surface texture and chemical interactions. The coating follows the exact contour of the support member, and when the implant changes its macroscopic dimension (e.g., volume or geometry), the coated biopolymer-based material and the synthetic polymer do not break, loosen, or fall off.

The support member can be made of a metal or a synthetic polymer. Examples of a support member include, but are not limited to, a stent (i.e., a tiny, expandable tube which holds heart arteries open following angioplasty), a coil (e.g., an embolization device), and a porous matrix such as a sponge (e.g., a hemostat or a microporous artificial blood vessel).
The invention also features a method of preparing an implant coated with a biopolymer-based material described above. The method involves providing a solvent (e.g., aqueous or nonaqueous) in which a biopolymer-based material is solubilized or dispersed; and spraying the solvent containing the biopolymer-based material onto a support member of an implant, the support member being dimensionally manipulatable. The biopolymer-based material is formed integrally on the surface of the support member. The method can further involves cross-linking the biopolymer-based material after the spraying step. Additionally, a solvent in which a synthetic polymer is solubilized or dispersed can be applied (e.g., by spraying or through chemical reactions) before or after the spraying step, thereby the synthetic polymer is formed integrally on an inner or outer surface of the biopolymer-based material.

The advantages of coating an implant with a biopolymer-based material include (1) resorbability and (2) no chronic foreign body reaction after the biopolymer-based material performs its intended function. The present invention provides a method of coating a biopolymer-based material onto the surface of a dimensionally manipulatable support member to form an implant that can be dimensionally manipulated without affecting its mechanical properties or its coating integrity. It is particularly useful for coating of flexible support members such as metal stents and coils, and support members with irregular surfaces such as porous metals, plastics, and biological form-like structures.

The details of one or more embodiments of the invention are set forth in the accompanying drawings and description below. Other features, objects, and advantages of the invention will be apparent from the drawings and the detailed description, and from the claims.

### BRIEF DESCRIPTION OF THE DRAWINGS

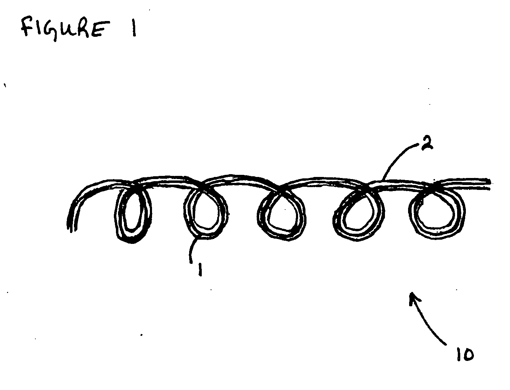

Figure 1 is a schematic drawing of a platinum coil coated with a type ] collagen-based material.

### DETAILED DESCRIPTION

The present invention relates to coating of support members with biopolymer-based materials for the modification of their surface properties without affecting their original biomechanical characteristics of the support members for their intended functions. The coated material follows the exact contour of the support member. The coating does not restrict the dimensional change of the support member, nor does it affect the physical integrity of the coated biopolymer-based material when the implant is subjected to a dimensional change. This is in contrast to the prior art coating methods which result in depositing a rigid film on the surface of a support member that restricts the dimensional change of the implant, and when the dimension of the implant changes, the coated film breaks.

Shown in Figure 1 is a coil (10) of the invention to be used for aneurysm embolization. The coil has a support member (1) and a coating (2).

The coating of the present invention involves preparation of a solvent-solubilized or dispersed biopolymer-based material, spraying the solvent-solubilized or dispersed biopolymer-based material onto the surface of a support member using a sprayer which deposits mist-like particles onto the exact contour surface of a support member so as not to affect the biomechanical characteristics of the support member. The coated implant can then be subjected to a chemical, physical, UV, or low dose gamma irradiation treatment to stabilize the surface adhered biopolymer-based material.

In one embodiment of the present invention, the biopolymer-based material is a collagen-based material. At the present time, at least 20 genetically distinct types of collagens have been discovered. Although any type of collagen and mixtures thereof can be used for the present invention, fiber-forming collagens (e.g., type I, type II and type III) are more preferred. In particular, type I collagen from human, animal or from genetically engineered methods is mostly preferred. Type I collagen can be easily isolated and purified from type I collagen-rich tissues such as skin, tendon, ligament, and bone of humans and animals. The methods of isolation and purification have been described in prior arts (see, e.g., Methods in Enzymology, vol. 82, pp. 33-64, 1982; The Preparation of Highly Purified Insoluble Collagen, Oneson, I., et al., Am. Leather Chemists Assoc., Vol. LXV, pp. 440-450, 1970; U.S. Patent No. 6,090,996). Genetically engineered collagens such as those marketed by Fibrogen (South San Francisco, CA) or from cell culture techniques such as those described by Advanced Tissue Sciences (La Jolla, CA) are also useful in this application. The methods of preparing a collagen-based solution or dispersion are described in the examples below.

In another embodiment of the present invention, the biopolymer-based material is a polysaccharide-based material. Cellulose materials from plants or bacteria, chitin or chitosan from shell fish, alginic acid from seaweeds, glycosaminoglycans from various sources can all be used for the present invention. Methods for the preparation of polysaccharide solution or dispersion are well known in the art (see, e.g., Chitin and Chitosan for Use as a Novel Biomedical Material, in "Advances in Biomedical Polymers," Hirano, et al., vol. 35, ed. C. G. Gebelein, pp. 285-297, Plenum Press, New York and London, 1987; Biological Gels: The Gelation of Chitosan and Chitin, Hirano, et al., in Biotechnology and Polymers, ed. C. G. Gebelein, pp. 181-188, Plenum Press, New York and London, 1991).

In another embodiment of the present invention, the biopolymer-based material is a mixture of collagen-based and polysaccharide-based material. Each of these materials has been described above.

In another embodiment of the present invention, the biopolymer-based material is a collagen-synthetic polymer composite. The synthetic polymers that are useful for the present invention include polyglycolic acid, polylactic acid, polyglycolic acid/poly-L-lactic acid copolymers, polycaprolactive, polyhydroxybutyrate/hydroxyvalerate copolymers, polydioxanone, polycarbonates, polyanhydrides, polytetrathalate, and polytetrafluoroethylene. The synthetic polymers may be grafted onto the surface of a biopolymer matrix by methods well known in the art, including plasma treatment, chemical modification and the like, or be a separate layer adhered to the surface of the biopolymer layer via physical, mechanical or physico-chemical interactions. See, e.g., A Collagen-Dacron Composite Vascular Graft for Arterial Reconstruction, in "Advances in Biomedical Polymers," Li, S-T, vol. 35, ed. C. G. Gebelein, pp. 171-183, Plenum Press, New York and London, 1987; and New Heparinizable Materials: Surface Grafting of Poly (Amide Amine) Chains on Polyurethane, Barbucci, R., et al., in "Biotechnology and Polymers," ed. C. G. Gebelein, pp. 259-276, Plenum Press, New York and London, 1991. Synthetic polymers may also be incorporated into the polysaccharide-based material similarly.

In another embodiment of the present invention, the biopolymer-based materials may optionally include bioactive agents for therapeutic applications. Bioactive materials that are suitable for the present invention include bioactive macromolecules (e.g., laminins, fibronectins, glycoproteins, nucleic acids, and mixtures thereof), drugs, alcohols, antibiotics, anti-proliferative (e.g., rapamycin), immunosuppressant agents, growth factors (e.g., fibroblast growth factors (FGFs), insulin-like growth factors (IGFs), platelet derived growth factors (PDGFs), epidermal growth factors (EGFs), transforming growth factors (TGFs), vascular endothelial growth factors (VEGFs), erythropoietin (EPO), and mixtures thereof), cytokines, and any other agents that have therapeutic applications.

The suitable sprayer for the present invention is any commercial sprayer or air brush or custom-designed sprayer that can produce mist-like particles using a compressed air technique. Depending on the nature of the support member, the size of the mist particle varies. For small and highly flexible support members such as stents and coils, the mist'particle should be fine and the coating follows exact contour of the support member and does not develop web-like strands that can cause adhesion of various parts of the support member affecting the biomechanical characteristics of the support member. The pressure of the compressed air, the nozzle size, the concentration and viscosity of the biopolymer-based material and the distance between the support member and the sprayer can be tested empirically to obtain an optimal condition for coating a specific support member. Generally, coating a metal stent is conducted at an air pressure of 10-60 psi, a nozzle size of 0.1-1.0 mm, the concentration of the biopolymer-based material from 0.1% to about 90% at a distance from the nozzle to the support member from about 6 to 24 inches.

Depending on the requirements of the coating, biopolymer-based materials may be coated from one to several layers of materials without affecting the biomechanical properties of the support member. This can be accomplished by controlling the spray parameters and the rotation or vibration of the support member at various speeds to provide centripetal or vibrational forces to minimize the potential web or film formation.

In another embodiment of the invention, the bioactive agent is dissolved or dispersed in the biopolymer-based material solution or dispersion. The biopolymer-based material solution or dispersion is then sprayed onto the support member.

In another embodiment of the invention, the bioactive agent can be sprayed (e.g., drug in solvent) or sprinkled (e.g., drug in powder form) on the biopolymer-based material-coated surface. Alternatively, the support member may be dipped in the bioactive agent solution to absorb the bioactive agent. Additional layer(s) of biopolymer-based material is then coated on the surface of the drug. This forms a sandwich-like configuration of the bioactive agent, and provides a mechanism for controlling the rate of release of the agent. Therefore, the rate of release of a bioactive agent can be controlled by the depth of the drug from the surface, the size of the bioactive agent, the hydrophilicty of the bioactive agent, the strength of physical and physical-chemical interaction between the bioactive agent and biopolymer-based material. By properly controlling these factors, a controlled release of a bioactive agent can be defined from the present invention.

The surface-coated implant can be treated with a chemical cross-linking agent to stabilize the biopolymer-based material. Any agent that interacts with hydroxyl, amino, guanidino and carboxyl groups can be used to stabilize the biopolymer-based materials (e.g., aldehyde compounds and carbodiimides). The cross-linking can also be accomplished with heat and vacuum, ultraviolet, low-dose gamma irradiation, and light-activated dye. Cross-linking methods are well known in the art.

The extent of cross-linking can be controlled by the concentration of the cross-linking agent, and the cross-linking time and temperature. The cross-linking condition can be optimized to control the in vivo stability of the coated biopolymer-based materials and also the rate of release of the incorporated bioactive agents. As an example, for stent coating, it is useful to maintain the coated material and the bioactive agent for at least 2-6 months to prevent the restonosis formation. Once the wound is healed, the biological process is stabilized for long-term efficacy of the implant.

The cross-linked implant can be subjected to a water rinse procedure to eliminate the cross-linking agent for biocompatibility. The rinsed implant is then dried either in the air or freeze-dried. Air-dry provides a compact structure of the coated material. If a bioactive agent is incorporated, it provides a slow release of the bioactive agent. The freeze-dried implant provides a more porous structure and therefore facilitates the release of the incorporated bioactive agent as well as the resorption of the coated biopolymer-based material due to the increased surface areas for biodegradation.

Generally, the method of coating a support member of the present invention can include the following steps:
(1) preparing a solvent-solubilized or dispersed biopolymer-based material,
(2) spraying the solvent-solubilized or dispersed biopolymer-based material onto the exact contour surface of a support member,
(3) stabilizing the surface-coated biopolymer-based material, and
(4) drying the surface-treated implant.

Even though the present invention describes the embodiments of coating of biopolymer-based materials on the support members, it is obvious to those skilled in the art that the invention can be easily adapted to any synthetic, biological, and a combination of synthetic and biological material thereof. Therefore, the spirit of the present invention encompasses a scope of applications beyond that described herein.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications recited herein are hereby incorporated by reference in their entirety.

### Preparation of Purified Type I Collagen

Bovine deep flexor tendons were obtained from an USDA approved abattoir. The fat and facia of tendon were removed and washed with water. The cleaned tendon was frozen and sliced into 0.5 mm slices with a meat slicer. Ten grams of sliced tendon was first extracted with 50 ml of water at room temperature for 24 hours. The extractant was discarded, and the tendon slices were extracted with 50 ml of 0.2 N HCl at room temperature for 24 hours. The acid solution was discarded, and 50 ml of water was added to the tendon to remove the residual acid. The rinsed slices were then extracted with 50 ml of 0.75 M NaOH at room temperature for 24 hours. The base solution was then discarded. The base-extracted slices were neutralized with 0.1 N HCI to pH 5 followed by several changes of water to remove the residual salt in the slices. The tendon was then defatted with 50 ml of isopropanol for 16 hours at room temperature. The extractant was decanted, and the slices were extracted with 50 ml of isopropanol for 24 hours at room temperature. The tendon was then dried under a clean hood.

### Preparation of Solvent-Solubilized Collagen

One gram of type I collagen thus prepared was dispersed in 1 L of 0.5 M acetic acid in the presence of 3 g pepsin and digested at 4°C for 24 hours. The material was filtered through a 100 mesh stainless steel mesh filter, and the solubilized collagen was precipitated with a 5% NaCl solution. The precipitated collagen was redissolved in 500 ml of 0.25 M acetic acid. The solution was filtered through a 100 mesh stainless steel mesh filter to eliminate the nonsolubilized particles. The collagen solution was then dialyzed with 2 L distilled water to remove the acid. The collagen solution was adjusted to a concentration of 0.1% until use.

### Preparation of Solvent-Dispersed Collagen

Six grams of type I collagen thus prepared was dispersed in 1 L of 0.07 M lactic acid, homogenized with a Silverson homogenizer (East Longmeadow, MA), and filtered through a 100 mesh stainless steel mesh filter. The dispersion was then de-aired under vacuum to remove the trapped air and stored at 4°C until use.

### Preparation of Solvent-Solubilized Polysaccharide

One gram of chitosan (Sigma, St. Louis, MO) was dissolved in 1 L of 0.25 M acetic acid solution, stirred and filtered through a 100 mesh stainless steel mesh filter to remove the particles. The dissolved chitosan was then dialyzed in 2 L of water to remove the acid and adjusted to a concentration of 0.1 % until use.

### Preparation of Solvent-Dispersed Collagen Containing Heparin

Three hundred (300) milligrams of sodium heparin (Celsus Laboratories, Cincinnati, OH) was dissolved in 1 L of 0.07 M lactic acid. Six grams of type 1 collagen thus prepared was then dispersed in the sodium heparin lactic acid solution, homogenized with a Silverson homogenizer (East Longmeadow, MA), and filtered through a 100 mesh stainless steel mesh filter. The dispersion was then de-aired under vacuum to remove the trapped air and stored at 4°C until use.

### Preparation of Platinum Coil Implant Coated with Collagen

One hundred (100) milliliters of the solvent-dispersed collagen was transferred into a container fitted for an air brush sprayer (Badger, Franklin Park, IL). The sprayer was then connected to a compressed air unit (Kaeser, Frederickberg, VA) attached to a regulator to control the air pressure. A platinum coil (Micrus Corp., Mountain View, CA) was fixed to a holder and lightly stretched to expose the surfaces for spraying. The coil was then rotated, and the solvent-dispersed collagen was sprayed on the coil. The coil was allowed to air-dry for 5 minutes. The spraying was repeated 2-3 times.

### Preparation of Platinum Coil Implant Coated with Collagen and Synthetic Polymer

The collagen-coated coil thus prepared was then sprayed with a 70% polyglycolic acid solution (Dupont Specialty Chemical, Wilmington, Delaware). The coil was allowed to air-dry for 1 hour. Alternatively, the collagen-coated coil thus prepared was reacted with a polyglycolic acid solution in the prescence of a carbodiimide compound (Sigma, St. Louis, MO) for 4-8 hours. The polyglycolic acid was covalently bonded onto the surface of the coated collagen implant.

### Preparation of Surface-Coated Metal Stent

One hundred (100) milliliters of solvent-dispersed collagen was transferred into a container fitted for an air brush sprayer (Badger, Franklin Park, IL). The sprayer was then connected to a compressed air unit (Kaeser, Frederickberg, VA) attached to a regulator to control the air pressure. A metal stent (Cook, Broomfield, CO) was fixed to a holder to expose the outer surfaces for spraying. While rotating the stent continuously, the solvent-dispersed collagen was sprayed on it. The stent was allowed to air-dry for 5 minutes. The collagen-coated stent was then immersed in a rapamycin solution (rapamycin dissolved in ethanol). The amount of rapamycin uptake was calculated to be about 200 µg. The rapamycin-coated stent was then sprayed with a second layer of collagen to stabilize the rapamycin. The implant was then air-dried.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. An implant comprising:
a support member, and
a biopolymer-based material formed integrally on a surface of the support member,
wherein the support member is dimensionally manipulatable, and the integrity of the biopolymer-based material is independent of dimensional changes in the support member.

2. The implant of claim 1, wherein the biopolymer-based material is protein-based.

3. The implant of claim 2, wherein the biopolymer-based material is collagen-based.

4. The implant of claim 3, wherein the support member is a stent, a coil or a sponge.

5. The implant of claim 1, wherein the biopolymer-based material is polysaccharide-based.

6. The implant of claim 5, wherein the biopolymer-based material is cellulose, chitosan, alginic acid, or glycosaminoglycan-based.

7. The implant of claim 6, wherein the support member is a stent, a coil or a sponge.

8. The implant of claim 1, wherein the biopolymer-based material is a mixture of a biopolymer and a synthetic polymer.

9. The implant of claim 8, wherein the support member is a stent, a coil or a sponge.

10. The implant of claim 1, further comprising a synthetic polymer formed integrally on an inner surface of the biopolymer-based material.

11. The implant of claim 10, wherein the support member is a stent, a coil or a sponge.

12. The implant of claim 1, further comprising a synthetic polymer formed integrally on an outer surface of the biopolymer-based material.

13. The implant of claim 12, wherein the support member is a stent, a coil or a sponge.

14. The implant of claim 1, wherein the support member is made of a metal.

15. The implant of claim 14, wherein the support member is a stent,a coil or a sponge.

16. The implant of claim 1, wherein the support member is made of a synthetic polymer.

17. The implant of claim 16, wherein the support member is a stent, a coil or a sponge.

18. The implant of claim 1, wherein the support member is a stent, a coil or a sponge.

19. The implant of claim 1, wherein the biopolymer-based material is admixed with a bioactive agent.

20. The implant of claim 12, wherein the synthetic polymer is admixed with a bioactive agent.

21. A method of preparing an implant coated with a biopolymer-based material, the method comprising:
providing a solvent in which a biopolymer-based material is solubilized or dispersed; and
spraying the solvent onto a support member of an implant, the support member being dimensionally manipulatable;
whereby the biopolymer-based material is formed integrally on a surface of the support member.

22. The method of claim 21, further comprising cross-linking the biopolymer-based material after the spraying step.

23. The method of claim 21, wherein the biopolymer-based material is a mixture of a biopolymer and a synthetic polymer.

24. The method of claim 21, before the spraying step, further comprising contacting the support member with a solvent in which a synthetic polymer is solubilized or dispersed, thereby the synthetic polymer is formed integrally on an inner surface of the biopolymer-based material.

25. The method of claim 21, after the spraying step, further comprising contacting the biopolymer-based material with a solvent in which a synthetic polymer is solubilized or dispersed, thereby the synthetic polymer is formed integrally on an outer surface of the biopolymer-based material.

26. An implant prepared according to the method of claim 21 or 22.
